# EUROPEAN PATENT APPLICATION

(11) **EP 4 209 992 A1**
(43) Date of publication of application: **12.07.2023**
(21) Application number: 21863604.1
(22) Date of filing: 31.08.2021
(51) Int. Cl.: G06T 7/00

(54) **AUTOMATIC IDENTIFICATION METHOD AND IDENTIFICATION SYSTEM FOR GASTROINTESTINAL MARKER**

(30) Priority: 01.09.2020 CN 202010902829
(71) Applicant: Ankon Technologies Co., Ltd, Wuhan, Hubei 430000 (CN)
(72) Inventor: HOU, Xiaohua, Wuhan, Hubei 430000 (CN); XIE, Xiaoping, Wuhan, Hubei 430000 (CN); JIN, Yu, Wuhan, Hubei 430000 (CN); WANG, Tingqi, Wuhan, Hubei 430000 (CN); GAO, Fei, Wuhan, Hubei 430000 (CN); DUAN, Xiaodong, Wuhan, Hubei 430000 (CN)
(74) Representative: Lavoix
(86) International application number: PCT/CN2021/115724
(87) International publication number: WO 2022/048540

(57) **Abstract**

An automatic identification method and an identification system for a gastrointestinal marker. The automatic identification method comprises the following steps: determining a suspected gastrointestinal marker region in an image; removing an overlapping suspected gastrointestinal marker region; and determining whether the suspected gastrointestinal marker region is part of the gastrointestinal marker. In the method, by means of processing and analyzing an image, positions of a gastrointestinal marker in the image can be automatically detected.

## Description

### CROSS-REFERENCE OF RELATED APPLICATIONS

The application claims priority from Chinese Patent Application No. 202010902829.6, filed September 1, 2020, entitled "Automatic Identification Method and Identification System for Gastrointestinal Marker", all of which are incorporated herein by reference in their entirety.

### FIELD OF INVENTION

The present invention relates to the field of medical device, and more particularly to an automatic identification method and an identification system capable of automatically detecting positions of gastrointestinal markers in an image.

### BACKGROUND

Examination of gastrointestinal motility by X-ray gastrointestinal markers is one of the important diagnostics means for gastrointestinal diseases. A gastrointestinal marker capsule is a capsule containing 20-24 thin slices of X-ray opaque gastrointestinal markers. An enclosure of the capsule can be naturally dissolved in the gastrointestinal tract. The gastrointestinal markers are made of biocompatible materials and can be configured to detect gastrointestinal transit time and are important diagnostic means for determining whether constipation or other gastrointestinal diseases exist.

At present, in a gastrointestinal motility examination, positions and types of the gastrointestinal markers need to be identified by a doctor according to a X-ray film taken, so that the workload of the doctor is greatly increased.

Therefore, it is necessary to provide an automatic identification method and an identification system for gastrointestinal markers to solve the above problem.

### SUMMARY OF THE INVENTION

The present invention provides an automatic identification method and an identification system for a gastrointestinal marker, which can automatically detect a position of the gastrointestinal marker in an image.

In order to achieve the above-mentioned objects of the present invention, the present invention uses the following technical solutions:
an automatic identification method for a gastrointestinal marker is provided, the method comprises:
determining a suspected gastrointestinal marker region in an image, comprising: segmenting the image by using a maximally stable extremal regions method, determining the suspected gastrointestinal marker region in the image; and calculating a minimum rectangle for each suspected gastrointestinal marker region in the image to form a first array;
removing an overlapping suspected gastrointestinal marker region, comprising: S3.1, creating an empty second array; S3.2, placing a rectangle with a maximum area into the second array, and removing the rectangle with the maximum area from the first array; S3.3, traversing the first array, calculating a ratio of intersection over union of a selected rectangle and the rectangle with the maximum area, and removing the selected rectangle from the first array when the ratio of intersection over union is greater than a threshold T1; repeating steps S3.2 and S3.3 for the first array until the first array is an empty array, and the second array finally formed is an array with the overlapping suspected gastrointestinal marker regions removed; and
determining whether the suspected gastrointestinal marker region is part of the gastrointestinal marker.

Further, the method further comprises improving identifiability of the gastrointestinal marker in the image by enhancing a contrast of the image.

Further, enhancing the contrast of the image comprises:
calculating a grayscale value range of the image, and obtaining a minimum grayscale value gₘᵢₙ and a maximum grayscale value gₘₐₓ; and
stretching grayscale values of the image to an interval of [0,255].

Further, in an image region R, a region maximum grayscale value is Max_R, a region minimum grayscale value is Min_R, and the suspected gastrointestinal marker region is a region in which a grayscale value is greater than a grayscale threshold T and a difference between the maximum grayscale value and the minimum grayscale value is less than a grayscale change threshold T_change; wherein a value range of the grayscale threshold T is: 150≥T≥200, and a value range of the grayscale change threshold T_change is: 10≥T_change≥20.

Further, whether the suspected gastrointestinal marker region is part of the gastrointestinal marker is determined according to a length of a longest side of each rectangle in the second array.

Further, L is defined as the length of the longest side of the suspected gastrointestinal marker region, and a pixel value range of L1 is: 30<L1≥40, a pixel value range of L2 is: 20<L2≥30, a pixel value range of L3 is: 10<L3≥20, M represents an error range coefficient of L, and a value range of M is: 1.0≥M≥1.2;
wherein it is determined that the suspected gastrointestinal marker region is not a gastrointestinal marker when L>L1*2 or L<L3*(M-1).

Further, a shape or a type of the gastrointestinal marker is determined according to the length of the longest side of the minimum rectangle of the suspected gastrointestinal marker region;
it is determined that the suspected gastrointestinal marker region is an overlapping of a plurality of gastrointestinal markers when L1*M<L<L1*2;
it is determined that the suspected gastrointestinal marker region is a tri-chamber gastrointestinal marker when (0.5*L1+0.5*L2)<L<L1*M;
it is determined that the suspected gastrointestinal marker region is an O-ring gastrointestinal marker when (0.5*L2+0.5*L3)<L<L2*M; and
it is determined that the suspected gastrointestinal marker region is a dot gastrointestinal marker when L3*(M-1)<L<L3*M.

It is another object of the present invention to provide an automatic identification system for the gastrointestinal marker, comprising:
a suspected gastrointestinal marker region identification module for determining a suspected gastrointestinal marker region in an image; wherein the suspected gastrointestinal marker region identification module comprises: a suspected gastrointestinal marker region determination module for segmenting the image by using a maximally stable extremal regions method to determine the suspected gastrointestinal marker region in the image; and a suspected gastrointestinal marker region labeling module for calculating a minimum rectangle of the suspected gastrointestinal marker region and forming a first array;
a de-overlapping module for removing overlapping suspected gastrointestinal marker regions; wherein the de-overlapping module comprises: a rectangle area obtaining module for obtaining areas of the rectangles in the first array; and an overlapping rectangle analysis and removal module for executing steps: S3.1, creating an empty second array; S3.2, placing a rectangle with a maximum area in the first array into the second array, and removing the rectangle with the maximum area from the first array; S3.3, traversing the first array, calculating a ratio of intersection over union of a selected rectangle and the rectangle with the maximum area, and removing the selected rectangle from the first array when the ratio of intersection over union is greater than a threshold T1; repeating steps S3.2 and S3.3 for the first array until the first array is an empty array, and the second array finally formed is an array with the overlapping suspected gastrointestinal marker regions removed; and
a gastrointestinal marker determination module for determining whether the suspected gastrointestinal marker region is part of the gastrointestinal marker.

Further, the automatic identification system for the gastrointestinal marker further comprises an image processing module for improving identifiability of the gastrointestinal marker in the image, where the image processing module comprises a grayscale value calculation module and an image contrast enhancement module;
the grayscale value calculation module for calculating a grayscale value range of the image and obtaining a minimum grayscale value gₘᵢₙ and a maximum grayscale value gₘₐₓ; and
the image contrast enhancement module for stretching grayscale values of the image to an interval of [0,255].

Further, the suspected gastrointestinal marker region determination module is configured to obtain a region maximum grayscale value Max_R and a region minimum grayscale value Min_R of an image region R, and the image region is considered as a suspected gastrointestinal marker region if meeting formulas: Min_R> T, and Max_R-Min_R <T_change; wherein a value range of a grayscale threshold T is: 150≥T≥200, and a value range of a grayscale change threshold T_change is: 10≥T_change≥20.

Further, the gastrointestinal marker determination module comprises:
a longest side obtaining module for obtaining a longest side of each rectangle in the second array;
a gastrointestinal marker judging module for determining whether the suspected gastrointestinal marker region is part of the gastrointestinal marker according to a length of the longest side; and the gastrointestinal marker determination module further comprises a gastrointestinal marker type judging module for determining which type of gastrointestinal marker the suspected gastrointestinal marker region belongs to according to the length of the longest side.

Further, the gastrointestinal marker judging module determining which type of gastrointestinal marker the suspected gastrointestinal marker region belongs to according to the length of the longest side comprises: L is defined as the length of the longest side of the suspected gastrointestinal marker region, and a pixel value range of L1 is: 30<L1≥40, a pixel value range of L2 is: 20<L2≥30, a pixel value range of L3 is: 10<L3≥20, M represents an error range coefficient of L, and a value range of M is: 1.0≥M≥1.2;it is determined that the suspected gastrointestinal marker region is not a gastrointestinal marker when L> L1*2 or L <L3*(M-1).

Further, the gastrointestinal marker judging module determining which type of gastrointestinal marker the suspected gastrointestinal marker region belongs to according to the length of the longest side further comprises: it is determined that the suspected gastrointestinal marker region is an overlapping of a plurality of gastrointestinal markers when L1*M<L<L1*2; it is determined that the suspected gastrointestinal marker region is a tri-chamber gastrointestinal marker when (0.5*L1+0.5*L2)<L<L1*M; it is determined that the suspected gastrointestinal marker region is an O-ring gastrointestinal marker when (0.5*L2+0.5*L3)<L<L2*M; and it is determined that the suspected gastrointestinal marker region is a dot gastrointestinal marker when L3*(M-1)<L<L3*M.

It is still another object of the present invention to provide an electronic device comprising a memory and a processor, wherein the memory stores a computer program that runs on the processor, and the processor executes the computer program to implement the steps of the image identification method as described above.

It is yet another object of the present invention to provide a computer-readable storage medium, wherein the computer-readable storage medium stores a computer program and the computer program is executed by the processor to implement the image identification method as described above.

According to all aspects of the present invention, the advantages over the prior art are that: by processing and analysis of an image, the automatic identification method for the gastrointestinal marker can automatically detect the position of a gastrointestinal marker in the image, determine the gastrointestinal motility, and thus greatly reduce the workload of a doctor.

### BRIEF DESCRIPTION OF THE DRAWINGS

In order to more clearly illustrate the technical solutions in the embodiments or prior art of the present invention, the accompanying drawings to be used in the description of the embodiments or prior art will be briefly described below. It will be apparent that the accompanying drawings in the following description are only embodiments of the present invention, and that other accompanying drawings may be obtained from the provided accompanying drawings without creative labor to those of ordinary skill in the art.
FIG. 1 is a flow schematic diagram of an automatic identification method for a gastrointestinal marker, in accordance with a preferred embodiment of the present invention.
FIG. 2 is a flow schematic diagram of the automatic identification method for the gastrointestinal marker, in accordance with another specific embodiment of the present invention.
FIG. 3 is a schematic diagram of a result from processing an original image in steps S1 and S2.
FIG. 4 is a schematic diagram of a result from processing in step S3 on the basis of FIG. 3.

### DETAILED DESCRIPTION

The present invention will be described in detail below with reference to the accompanying drawings and preferred embodiments. However, the embodiments are not intended to limit the invention, and the structural, method, or functional changes made by those skilled in the art in accordance with the embodiments are comprised in the scope of the present invention.

The present invention provides an automatic identification method for a gastrointestinal marker, which is used for identifying a position of the gastrointestinal marker in an image. The gastrointestinal marker can be an X-ray contrast agent such as barium sulfate, bismuth salt or tungsten body in the prior art, with a shape and structure not limited; and can also be a newly developed gastrointestinal marker.

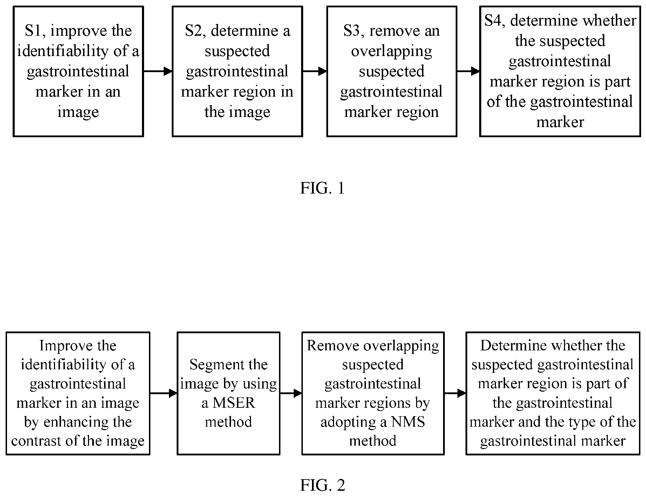

Referring to FIGS. 1-4, the automatic identification method for the gastrointestinal marker mainly comprises the steps: step S1, improving the identifiability of the gastrointestinal marker in the image, so as to rapidly determine a suspected gastrointestinal marker region; step S2, determining the suspected gastrointestinal marker region in the image; step S3, removing an overlapping suspected gastrointestinal marker region; and step S4, determining whether the suspected gastrointestinal marker region is part of the gastrointestinal marker. By image processing, the method can automatically detect a position of the gastrointestinal marker in the image, determine the gastrointestinal motility, and greatly reduce the workload of doctors.

In step S1, the identifiability of the gastrointestinal marker in the image is improved by enhancing the contrast of the image. That is, a captured original image is enhanced to improve the identifiability of the gastrointestinal marker in the image.

Specifically, step S1 comprises the following steps: step S1.1, calculating a grayscale value range of the image, and obtaining a minimum grayscale value gₘᵢₙ and a maximum grayscale value gₘₐₓ; and step S1.2, stretching the grayscale value of the image to an interval of [0,255], to enhance the identifiability of the gastrointestinal marker in the image.

When the identifiability of the gastrointestinal marker in the image is high and the requirements of the subsequent steps are met, step S1 may be omitted.

In step S2, the image is segmented by using a maximally stable extremal regions (MSER) method, the suspected gastrointestinal marker region is determined in the image, and a minimum rectangle of the suspected gastrointestinal marker region is calculated. In an X-ray image, the gastrointestinal marker region is a region where the grayscale value does not vary much and is higher than the background. In an image region R, the region maximum grayscale value is Max_R, the region minimum grayscale value is Min_R, and the suspected gastrointestinal marker region is defined as a region in which the grayscale value is greater than a grayscale threshold T and the difference between the maximum grayscale value and the minimum grayscale value is less than a grayscale change threshold T_change. The image region can be considered as a suspected gastrointestinal marker region if meeting the following formulas: Min_R> T, and Max_R-Min_R <T_change. Thus, using the MSER method, a plurality of suspected gastrointestinal marker regions can be found. Further, step S2 further comprises: calculating a minimum rectangle of the suspected gastrointestinal marker region in the image, resulting in forming a first array (rectangle array rectangles[]).

In a specific embodiment, the value range of the grayscale threshold T is: 150≥T≥200, and the value range of the grayscale change threshold T_change is: 10≥T_change≥20.

A plurality of suspected gastrointestinal marker regions obtained in step S2 may overlap, and in step S3, a non-maximum suppression (NMS) method is adopted to remove the overlapping suspected gastrointestinal marker regions. According to the present invention, the NMS method is used to suppress redundant regions, and the suppression process is an iteration-traversal-elimination process.

Specifically, the step S3 comprises the following steps: step S3.1, creating an empty second array (rectangles_keep[]); step S3.2, sorting the rectangles in the first array according to the areas from largest to smallest, where the sorting process is not a necessary step and can also be omitted; recording the rectangle with the maximum area (rectangle_max) as a first rectangle and putting into the second array, and correspondingly, removing the rectangle with the maximum area from the first array; step S3.3, traversing the first array, calculating a ratio of intersection over union (IoU) of the selected rectangle (rectangle_select) and the rectangle with the maximum area, and removing the selected rectangle from the first array when the ratio of IoU is greater than a certain threshold T1. The threshold T1 may be defined according to actual requirements.

That is, in step S3.3, the ratios of IoU of the remaining rectangles in the first array and the first rectangle placed in the second array are sequentially calculated, and the corresponding rectangles with the ratios of IoU greater than T1 are removed, so as to form a new first array.

Steps S3.2 and S3.3 are then repeated for the new first array. Specifically, in step S3.2, the rectangle with the maximum area in the new first array is recorded as a second rectangle and put into the second array, and the second rectangle is correspondingly removed from the new first array. Therefore, the second array contains two rectangles: the first rectangle and the second rectangle. In step S3.3, the ratios of IoU of the remaining rectangles in the new first array and the second rectangle placed in the second array are sequentially calculated, and the corresponding rectangles with the ratios of IoU greater than T1 are removed, so as to form another new first array.

In this way, during traversing, the number of rectangles in the first array decreases continuously until it becomes an empty array; while the number of rectangles in the second array increases continuously, and the second array finally formed is the array formed after the overlapping suspected gastrointestinal marker regions are removed.

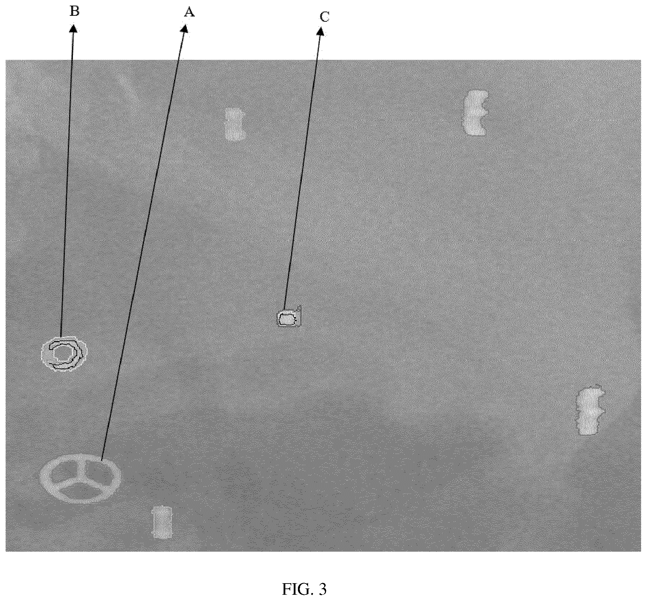

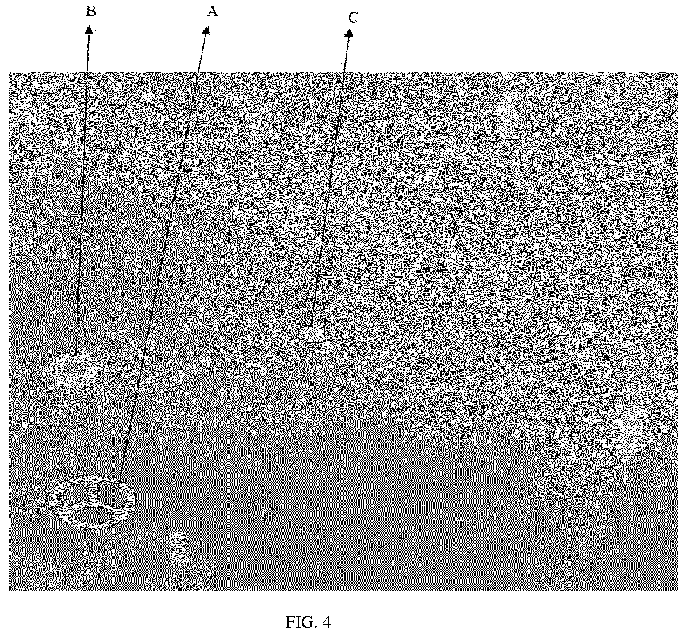

As can be seen from the comparison between FIG. 3 and FIG. 4, after the above steps, the overlapping suspected gastrointestinal marker regions at B and C are filtered out.

Referring to FIGS. 3 and 4, in a specific embodiment, there are three suspected gastrointestinal marker regions, labeled A, B, and C. After the steps S1 and S2, the regions B and C in FIG. 3 have two region frames representing the suspected gastrointestinal marker regions, which represent the overlapping suspected gastrointestinal marker regions. Then, after step S3, the overlapping suspected gastrointestinal marker regions of the regions B and C in FIG. 4 are removed, that is, only one region frame representing the suspected gastrointestinal marker region remains, representing the suspected gastrointestinal marker region from which the overlapping region is removed. It is convenient to accurately identify whether suspected gastrointestinal mark region is part of the gastrointestinal marker and the type thereof. It should be noted that the region frames in FIG. 3 and FIG. 4 represent the actual suspected gastrointestinal marker region, while the rectangle described above is the smallest rectangle that can cover the actual suspected gastrointestinal marker region during the above identification method, and is not shown in FIGS.

Step S4 determines whether the suspected gastrointestinal marker region is part of the gastrointestinal marker. Specifically, whether the suspected gastrointestinal marker region is part of the gastrointestinal marker is determined according to the length of the longest side of each rectangle in the second array.

According to the present invention, L is defined as the length of the longest side of the suspected gastrointestinal marker region, and the pixel value range of L1 is: 30<L1≥40, the pixel value range of L2 is: 20<L2≥30, the pixel value range of L3 is: 10<L3≥20, M represents the error range coefficient of L, and the value range of M is: 1.0≥M≥1.2. If L>L1*2 or L<L3*(M-1), the suspected gastrointestinal marker region is not a gastrointestinal marker.

Further, the shape or the type of the gastrointestinal marker can be determined according to the length of the longest side of the minimum rectangle of the suspected gastrointestinal marker region. Specifically, if L1*M<L<L1*2, the suspected gastrointestinal marker region is an overlapping of a plurality of gastrointestinal markers; if (0.5*L1+0.5*L2)<L<L1*M, the suspected gastrointestinal marker region is a tri-chamber gastrointestinal marker; if (0.5*L2+0.5*L3)<L<L2*M, the suspected gastrointestinal marker region is an O-ring gastrointestinal marker; and if L3*(M-1)<L<L3*M, the suspected gastrointestinal marker region is a dot gastrointestinal marker.

The present invention further provides an automatic identification system for the gastrointestinal marker, comprising:
a suspected gastrointestinal marker region identification module for determining a suspected gastrointestinal marker region in the image;
a de-overlapping module for removing overlapping suspected gastrointestinal marker regions; and
a gastrointestinal marker determination module for determining whether the suspected gastrointestinal marker region is part of the gastrointestinal marker.

Further, the automatic identification system for the gastrointestinal marker further comprises an image processing module for improving the identifiability of the gastrointestinal marker in the image, where the image processing module comprises a grayscale value calculation module and an image contrast enhancement module. The grayscale value calculation module is configured for calculating a grayscale value range of the image and obtaining a minimum grayscale value gₘᵢₙ and a maximum grayscale value gₘₐₓ. The image contrast enhancement module is configured for stretching the grayscale value of the image to an interval of [0, 255]. The image processing module for improving the identifiability of the gastrointestinal marker in the image can be referred to step S 1 above.

When the identifiability of the gastrointestinal marker in the image is relatively high and meets the requirements of the subsequent steps, the automatic identification system may not necessarily comprise the image processing module.

The suspected gastrointestinal marker region identification module comprises:
a suspected gastrointestinal marker region determination module, is configured to segment the image by using a maximally stable extremal regions (MSER) method and determines the suspected gastrointestinal marker region in the image. Specifically, the suspected gastrointestinal marker region determination module is configured to obtain a region maximum grayscale value Max_R and a region minimum grayscale value Min_R of the image region R, and the image region is considered as a suspected gastrointestinal marker region if meeting the following formulas: Min_R> T, and Max_R-Min_R <T_change; wherein the value range of the grayscale threshold T is: 150≥T≥200, and the value range of the grayscale change threshold T_change is: 10≥T_change≥20.And
a suspected gastrointestinal marker region labeling module, is configured for calculating a minimum rectangle of the suspected gastrointestinal marker region and forming a first array.

The suspected gastrointestinal marker region identification module for determining the suspected gastrointestinal marker region in the image can be referred to above step S2.

The de-overlapping module comprises: a rectangle area obtaining module, is configured for obtaining the areas of the rectangles in the first array; an overlapping rectangle analysis and removal module, is configured for creating an empty second array, placing the rectangle with the maximum area in the first array into the second array, correspondingly, removing the rectangle with the maximum area from the first array. The overlapping rectangle analysis and removal module is further configured for traversing the first array, calculating a ratio of intersection over union (IoU) of the selected rectangle and the rectangle with the maximum area, and removing the selected rectangle from the first array when the ratio of IoU is greater than a certain threshold T1. Specifically, the method of the de-overlapping module for removing the overlapping suspected gastrointestinal marker regions can be referred to above step S3.

The gastrointestinal marker determination module comprises: a longest side obtaining module, is configured for obtaining the longest side of each rectangle in the second array; and a gastrointestinal marker judging module, is configured for determining whether the suspected gastrointestinal marker region is part of the gastrointestinal marker according to the length of the longest side.

Further, the gastrointestinal marker determination module further comprises a gastrointestinal marker type judging module configured for determining which type of gastrointestinal marker the suspected gastrointestinal marker region belongs to according to the length of the longest side.

Further, the process of the gastrointestinal marker judging module determining which type of gastrointestinal marker the suspected gastrointestinal marker region belongs to according to the length of the longest side comprises: defining L as the length of the longest side of the suspected gastrointestinal marker region, and the pixel value range of L1 is: 30<L1≥40, the pixel value range of L2 is: 20<L2≥30, and the pixel value range of L3 is: 10<L3≥20, M represents the error range coefficient of L, and the value range of M is:1.0≥M≥1.2; if L> L1*2 or L <L3*(M-1), determining that the suspected gastrointestinal marker region is not a gastrointestinal marker.

Further, the process of the gastrointestinal marker judging module determining which type of gastrointestinal marker the suspected gastrointestinal marker region belongs to according to the length of the longest side further comprises: if L1*M<L<L1*2, determining that the suspected gastrointestinal marker region is an overlapping of a plurality of gastrointestinal markers; if (0.5*L1+0.5*L2)<L<L1*M, determining that the suspected gastrointestinal marker region is a tri-chamber gastrointestinal marker; if (0.5*L2+0.5*L3)<L<L2*M, determining that the suspected gastrointestinal marker region is an O-ring gastrointestinal marker; and if L3*(M-1)<L<L3*M, determining that the suspected gastrointestinal marker region is a dot gastrointestinal marker.

Specifically, the method of the gastrointestinal marker determination module determining the gastrointestinal marker can be referred to the step S4 of the above automatic identification method for the gastrointestinal marker, which is not described here.

The present invention further provides an electronic device comprising a memory and a processor, wherein the memory stores a computer program that can run on the processor, and the processor executes the computer program to implement the steps of the image identification method as described above.

The present invention further provides a computer-readable storage medium, wherein the computer-readable storage medium stores a computer program and the computer program is executed by the processor to implement the image identification method as described above.
In summary, the automatic identification method for gastrointestinal markers of the present invention can automatically detect the positions and types of gastrointestinal markers in the image, so as to identify the positions of different types of gastrointestinal markers in the gastrointestinal tract and determine the gastrointestinal motility of a subject.

It should be understood that, although the description is described in terms of embodiments, not every embodiment merely comprises an independent technical solution. The description is presented in this way only for the sake of clarity, those skilled in the art should have the description as a whole, and the technical solutions in each embodiment may also be combined as appropriate to form other embodiments that can be understood by those skilled in the art.

The series of detailed descriptions set forth above are only specific descriptions of feasible embodiments of the present invention and are not intended to limit the scope of protection of the present invention. Equivalent embodiments or modifications made within the spirit of the present invention should be included within the protection scope of the present invention.

## Claims

1. An automatic identification method for a gastrointestinal marker, comprising:
determining a suspected gastrointestinal marker region in an image, comprising:
segmenting the image by using a maximally stable extremal regions method, determining the suspected gastrointestinal marker region in the image; and calculating a minimum rectangle for each suspected gastrointestinal marker region in the image to form a first array;
removing an overlapping suspected gastrointestinal marker region, comprising: S3.1,
creating an empty second array; S3.2, placing a rectangle with a maximum area into the second array, and removing the rectangle with the maximum area from the first array; S3.3,
traversing the first array, calculating a ratio of intersection over union of a selected rectangle and the rectangle with the maximum area, and removing the selected rectangle from the first array when the ratio of intersection over union is greater than a threshold T1;
repeating steps S3.2 and S3.3 for the first array until the first array is an empty array, and
the second array finally formed is an array with the overlapping suspected gastrointestinal marker regions removed; and
determining whether the suspected gastrointestinal marker region is part of the gastrointestinal marker.

2. The automatic identification method of claim 1, further comprising: improving identifiability of the gastrointestinal marker in the image by enhancing a contrast of the image.

3. The automatic identification method of claim 2, wherein enhancing the contrast of the image comprises:
calculating a grayscale value range of the image, and obtaining a minimum grayscale value gₘᵢₙ and a maximum grayscale value gₘₐₓ; and
stretching grayscale values of the image to an interval of [0,255].

4. The automatic identification method of claim 1, wherein in an image region R, a region maximum grayscale value is Max_R, a region minimum grayscale value is Min_R, and the suspected gastrointestinal marker region is a region in which a grayscale value is greater than a grayscale threshold T and a difference between the maximum grayscale value and the minimum grayscale value is less than a grayscale change threshold T_change; wherein a value range of the grayscale threshold T is: 150≥T≥200, and a value range of the grayscale change threshold T_change is: 10≥T_change≥20.

5. The automatic identification method of claim 1, wherein whether the suspected gastrointestinal marker region is part of the gastrointestinal marker is determined according to a length of a longest side of each rectangle in the second array.

6. The automatic identification method of claim 5, wherein L is defined as the length of the longest side of the suspected gastrointestinal marker region, and a pixel value range of L1 is: 30<L1≥40, a pixel value range of L2 is: 20<L2≥30, a pixel value range of L3 is:
10<L3≥20, M represents an error range coefficient of L, and a value range of M is:
1.0≥M≥1.2; wherein it is determined that the suspected gastrointestinal marker region is not a gastrointestinal marker when L>L1*2 or L<L3*(M-1).

7. The automatic identification method of claim 6, wherein a shape or a type of the gastrointestinal marker is determined according to the length of the longest side of the minimum rectangle of the suspected gastrointestinal marker region;
it is determined that the suspected gastrointestinal marker region is an overlapping of a plurality of gastrointestinal markers when L1*M<L<L1*2;
it is determined that the suspected gastrointestinal marker region is a tri-chamber gastrointestinal marker when (0.5*L1+0.5*L2)<L<L1*M;
it is determined that the suspected gastrointestinal marker region is an O-ring gastrointestinal marker when (0.5*L2+0.5*L3)<L<L2*M; and
it is determined that the suspected gastrointestinal marker region is a dot gastrointestinal marker when L3*(M-1)<L<L3*M.

8. An automatic identification system for a gastrointestinal marker, comprising:
a suspected gastrointestinal marker region identification module for determining a suspected gastrointestinal marker region in an image; wherein the suspected gastrointestinal marker region identification module comprises: a suspected gastrointestinal marker region determination module for segmenting the image by using a maximally stable extremal regions method to determine the suspected gastrointestinal marker region in the image; and a suspected gastrointestinal marker region labeling module for calculating a minimum rectangle of the suspected gastrointestinal marker region and forming a first array;
a de-overlapping module for removing overlapping suspected gastrointestinal marker regions; wherein the de-overlapping module comprises: a rectangle area obtaining module for obtaining areas of the rectangles in the first array; and an overlapping rectangle analysis and removal module for executing steps: S3.1, creating an empty second array; S3.2, placing a rectangle with a maximum area in the first array into the second array, and
removing the rectangle with the maximum area from the first array; S3.3, traversing the first array, calculating a ratio of intersection over union of a selected rectangle and the rectangle with the maximum area, and removing the selected rectangle from the first array when the ratio of intersection over union is greater than a threshold T1; repeating steps S3.2 and S3.3 for the first array until the first array is an empty array, and the second array finally formed is an array with the overlapping suspected gastrointestinal marker regions removed; and
a gastrointestinal marker determination module for determining whether the suspected gastrointestinal marker region is part of the gastrointestinal marker.

9. The automatic identification system of claim 8, wherein the automatic identification system further comprises an image processing module for improving identifiability of the gastrointestinal marker in the image, wherein the image processing module comprises:
a grayscale value calculation module for calculating a grayscale value range of the image and obtaining a minimum grayscale value gₘᵢₙ and a maximum grayscale value gₘₐₓ; and
an image contrast enhancement module for stretching grayscale values of the image to an interval of [0,255].

10. The automatic identification system of claim 8, wherein the suspected gastrointestinal marker region determination module is configured to obtain a region maximum grayscale value Max_R and a region minimum grayscale value Min_R of an image region R, and the image region is considered as a suspected gastrointestinal marker region if meeting formulas: Min_R> T, and Max_R-Min_R <T_change; wherein a value range of a grayscale threshold T is: 150≥T≥200, and a value range of a grayscale change threshold T_change is: 10≥T_change≥20.

11. The automatic identification system of claim 8, wherein the gastrointestinal marker determination module comprises:
a longest side obtaining module for obtaining a longest side of each rectangle in the second array;
a gastrointestinal marker judging module for determining whether the suspected gastrointestinal marker region is part of the gastrointestinal marker according to a length of the longest side; and the gastrointestinal marker determination module further comprises a gastrointestinal marker type judging module for determining which type of gastrointestinal marker the suspected gastrointestinal marker region belongs to according to the length of the longest side.

12. The automatic identification system of claim 11, wherein the gastrointestinal marker judging module determining which type of gastrointestinal marker the suspected gastrointestinal marker region belongs to according to the length of the longest side comprises: L is defined as the length of the longest side of the suspected gastrointestinal marker region, and a pixel value range of L1 is: 30<L1≥40, a pixel value range of L2 is: 20<L2≥30, a pixel value range of L3 is: 10<L3≥20, M represents an error range coefficient of L, and a value range of M is: 1.0≥M≥1.2;it is determined that the suspected gastrointestinal marker region is not a gastrointestinal marker when L> L1*2 or L <L3*(M-1).

13. The automatic identification system of claim 12, wherein the gastrointestinal marker judging module determining which type of gastrointestinal marker the suspected gastrointestinal marker region belongs to according to the length of the longest side further comprises: it is determined that the suspected gastrointestinal marker region is an overlapping of a plurality of gastrointestinal markers when L1*M<L<L1*2; it is determined that the suspected gastrointestinal marker region is a tri-chamber gastrointestinal marker when (0.5*L1+0.5*L2)<L<L1*M; it is determined that the suspected gastrointestinal marker region is an O-ring gastrointestinal marker when (0.5*L2+0.5*L3)<L<L2*M; and it is determined that the suspected gastrointestinal marker region is a dot gastrointestinal marker when L3*(M-1)<L<L3*M.

14. An electronic device, comprising a memory and a processor, wherein the memory stores a computer program that runs on the processor, and the processor executes the computer program to implement the steps in the image identification method of claim 1.

15. A computer-readable storage medium having stored thereon a computer program, wherein the computer program is executed by a processor to implement the image identification method of claim 1.
